# EUROPEAN PATENT SPECIFICATION

(11) **EP 3 967 702 B1**
(45) Date of publication and mention of the grant of the patent: **20.03.2024**
(21) Application number: 20805780.2
(22) Date of filing: 09.05.2020
(51) Int. Cl.: C07K 5/062, C07D 403/06, A61K 31/4045, A61P 35/00, A61K 38/00

(54) **CRYSTALLIZATION OF SMAC MIMIC USED AS IAP INHIBITOR AND PREPARATION METHOD THEREOF**
KRISTALLISATION VON SMAC-MIMIC ALS IAP-INHIBITOR UND VERFAHREN ZU DESSEN HERSTELLUNG
CRISTALLISATION D'UN MIMÉTIQUE DE SMAC UTILISÉ EN TANT QU'INHIBITEUR D'IAP ET SON PROCÉDÉ DE PRÉPARATION

(30) Priority: 10.05.2019 CN 201910389970
(43) Date of publication of application: 16.03.2022
(73) Proprietor: CHIA TAI TIANQING PHARMACEUTICAL GROUP CO., LTD., Lianyungang, Jiangsu 222062 (CN)
(72) Inventor: LIU, Yingchun, Nanjing, Jiangsu 210032 (CN); XU, Zhaobing, Nanjing, Jiangsu 210032 (CN); HU, Lihong, Nanjing, Jiangsu 210032 (CN); DING, Charles Z., Nanjing, Jiangsu 210032 (CN); ZHU, Xingxun, Nanjing, Jiangsu 210032 (CN); CHEN, Shuhui, Nanjing, Jiangsu 210032 (CN)
(74) Representative: Grünecker Patent- und Rechtsanwälte PartG mbB
(86) International application number: PCT/CN2020/089437
(87) International publication number: WO 2020/228642

(56) References cited:
- WO-A1-2019/091492
- WO-A2-2008/014252
- CN-A- 101 035 802
- CN-A- 101 146 803
- CN-A- 101 595 121
- CN-A- 101 605 786
- CN-A- 101 951 766
- SARABIA, F . et al.: "Stereoselective synthesis of E-64 and related cysteine proteases inhibitors from 2, 3-epoxyamides", Bioorganic & Medicinal Chemistry, vol. 13, 12 January 2005 (2005-01-12), pages 1691-1705, XP004736081, DOI: 10.1016/j.bmc.2004.12.018

## Description

### CROSS-REFERENCE TO RELATED APPLICATIONS

The present application claims priority and benefit to the Chinese Patent Application No. 201910389970.8, filed with National Intellectual Property Administration, PRC on May 10, 2019.

### TECHNICAL FIELD

The present application relates to the field of pharmaceutical chemistry, and in particular, to a crystalline form of an SMAC mimetic as an IAP inhibitor and a method for preparing the same, as well as use of the crystalline form in preparing a medicament for treating a cancer that can benefit from cIAP1 inhibition.

### BACKGROUND

Programmed cell death plays a key role in regulating cell number and eliminating stressed or damaged cells from normal tissues. Indeed, apoptotic signaling network mechanisms inherent in most cells provide a major barrier against the development and progression of cancers in human. However, cancer cells share in common that they cannot initiate an apoptotic program and lack the appropriate apoptosis due to loss of normal apoptotic mechanisms. Most anticancer therapies today, including chemotherapy, radiation therapy and immunotherapy, act by indirectly inducing apoptosis in cancer cells. Thus, failure in initiating apoptotic programs due to defects in normal apoptotic mechanisms in cancer cells is usually associated with increased resistance to chemotherapy, radiation therapy or immunotherapy-induced apoptosis. Therefore, targeting key negative regulators that play an important role in directly suppressing cancer cell apoptosis would be a very promising therapeutic strategy for new anticancer drug design.

Two important classes of negative regulators of apoptosis have been identified. The first class of regulatory factors are the Bcl-2 family proteins, for example, two potent anti-apoptotic proteins, Bcl-2 and Bcl-XL.

The second class of negative regulators for apoptosis are inhibitors of apoptosis proteins (IAPs). IAPs were first discovered in baculoviruses by their functional ability to substitute for P35 protein. Such proteins include: XIAP, cIAP1, cIAP2, ML-IAP, ILP-2, NAIP, apollon, and survivin. Among these, X-linked inhibitor of apoptosis (XIAP) suppresses apoptosis by directly inhibiting caspase-3, caspase-7 and caspase-9. cIAPs mainly inhibit apoptosis by blocking death receptor pathways. When cIAPs are degraded, the substrate NIK (NF-xB-inducing kinase) escapes from degradation, and thus accumulates and activates NF-κB through a non-classical pathway. Activated NF-κB promotes the secretion of TNFα, which binds to TNF-R1 (TNF receptor 1) to initiate the death receptor pathway. Due to the degradation of cIAPs, RIPK1 (receptor interacting protein kinase 1) exhibits increased secretion and forms pro-apoptotic complex RIPK1-FADD-caspase-8 with FADD (Fas-associated death domin) and caspase-8, which then activates caspase-3 and initiates apoptosis.

Overexpression of cIAP1 and cIAP2 due to frequent chromosomal amplification in the 11q21-q23 region, which encompasses two genes, was observed in a variety of malignant diseases including neuroblastoma, renal cell carcinoma, colorectal cancer and gastric cancer.

### BRIEF SUMMARY

In one aspect, the present application provides a crystalline form of a compound of formula (I),

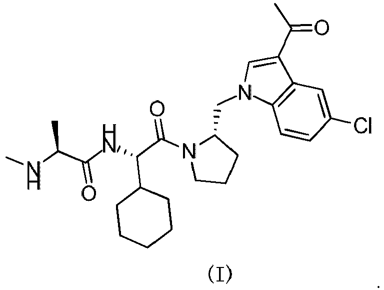

In another aspect, the present application provides a crystalline composition of the compound of formula (I), comprising 50% or more, preferably 75% or more, more preferably 90% or more, and most preferably 95% or more by weight of the crystalline form of the compound of formula (I).

In another aspect, the present application provides a pharmaceutical composition, comprising a therapeutically effective amount of the crystalline form of the compound of formula (I), or the crystalline composition of the compound of formula (I); the pharmaceutical composition may further comprise at least one pharmaceutically acceptable carrier or other excipient.

In another aspect, the present application provides use of the crystalline form of the compound of formula (I), the crystalline composition of the compound of formula (I), or the pharmaceutical composition as described above in preparing a medicament for treating a cancer that can benefit from cIAP1 inhibition.

In another aspect, the present application provides the crystalline form of the compound of formula (I), the crystalline composition of the compound of formula (I), or the pharmaceutical composition as described above, for use in treating a cancer that can benefit from cIAP1 inhibition in a mammal.

### SUMMARY

One aspect of the present application is to provide a crystalline form of a compound of formula (I),

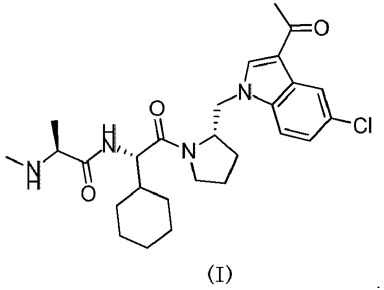

The crystalline form described herein may be present in the form of a non-solvate or a solvate, for example, a hydrate.

In one embodiment of the present application, the crystalline form of the compound of formula (I) is crystalline form A having diffraction peaks at the following 2θ in an X-ray powder diffraction pattern using Cu-Kα radiation: 12.1°±0.200°, 16.1°±0.200°, 18.5°±0.200°, 20.2°±0.200°, 21.3°±0.200° and 23.0°±0.200°.

In one embodiment of the present application, crystalline form A has diffraction peaks at the following 2θ in an X-ray powder diffraction pattern using Cu-Kα radiation: 12.1°±0.200°, 16.1°±0.200°, 18.5°±0.200°, 18.8°±0.200°, 19.2°±0.200°, 19.8°±0.200°, 20.2°±0.200°, 21.3°±0.200°, 23.0°±0.200°, 26.6°±0.200° and 27.4°±0.200°.

In one embodiment of the present application, crystalline form A has diffraction peaks at the following 2θ in an X-ray powder diffraction pattern using Cu-Kα radiation: 7.0°±0.200°, 8.7°±0.200°, 12.1°±0.200°, 13.2°±0.200°, 13.9°±0.200°, 16.1°±0.200°, 16.7°±0.200°, 18.5°±0.200°, 18.8°±0.200°, 19.2°±0.200°, 19.8°±0.200°, 20.2°±0.200°, 21.0°±0.200°, 21.3°±0.200°, 23.0°±0.200°, 24.3°±0.200°, 25.3°±0.200°, 26.6°±0.200° and 27.4°±0.200°.

In one embodiment of the present application, crystalline form A has diffraction peaks at the following 2θ in an X-ray powder diffraction pattern using Cu-Kα radiation: 7.0°±0.200°, 8.7°±0.200°, 12.1°±0.200°, 13.2°±0.200°, 13.9°±0.200°, 16.1°±0.200°, 16.5°±0.200°, 16.7°±0.200°, 18.5°±0.200°, 18.8°±0.200°, 19.2°±0.200°, 19.8°±0.200°, 20.2°±0.200°, 21.0°±0.200°, 21.3°±0.200°, 23.0°±0.200°, 23.2°±0.200°, 24.3°±0.200°, 25.3°±0.200°, 26.6°±0.200°, 26.9°±0.200°, 27.4°±0.200° and 29.4°±0.200°.

In one embodiment of the present application, crystalline form A has diffraction peaks at the following 2θ in an X-ray powder diffraction pattern using Cu-Kα radiation: 7.0°±0.200°, 8.7°±0.200°, 9.7°±0.200°, 10.6°±0.200°, 11.4°±0.200°, 12.1°±0.200°, 13.2°±0.200°, 13.9°±0.200°, 16.1°±0.200°, 16.5°±0.200°, 16.7°±0.200°, 17.5°±0.200°, 18.5°±0.200°, 18.8°±0.200°, 19.2°±0.200°, 19.5°±0.200°, 19.8°±0.200°, 20.2°±0.200°, 21.0°±0.200°, 21.3°±0.200°, 22.5°±0.200°, 23.0°±0.200°, 23.2°±0.200°, 23.8°±0.200°, 24.3°±0.200°, 24.6°±0.200°, 25.3°±0.200°, 25.8°±0.200°, 26.6°±0.200°, 26.9°±0.200°, 27.4°±0.200°, 28.1°±0.200°, 29.1°±0.200°, 29.4°±0.200°, 30.1°±0.200°, 30.6°±0.200°, 30.8°±0.200°, 31.3°±0.200°, 31.8°±0.200°, 32.2°±0.200°, 32.8°±0.200°, 33.7°±0.200°, 34.0°±0.200°, 34.8°±0.200°, 35.5°±0.200°, 36.6°±0.200°, 37.6°±0.200°, 38.6°±0.200° and 39.6°±0.200°.

In one embodiment of the present application, positions and intensities of the diffraction peaks in an X-ray powder diffraction pattern using Cu-Kα radiation for crystalline form A of the compound of formula (I) disclosed herein are shown in Table 1:

**Table 1. XRPD pattern characterization data for crystalline form A**

| No. | 2θ (°) | Relative intensity (%) | No. | 2θ (°) | Relative intensity (%) |
|---|---|---|---|---|---|
| 1 | 7.0 | 8.2 | 26 | 24.6 | 7.4 |
| 2 | 8.7 | 6.0 | 27 | 25.3 | 10.9 |
| 3 | 9.7 | 1.5 | 28 | 25.8 | 1.5 |
| 4 | 10.6 | 3.9 | 29 | 26.6 | 20.3 |
| 5 | 11.4 | 2.9 | 30 | 26.9 | 9.9 |
| 6 | 12.1 | 49.6 | 31 | 27.4 | 21.4 |
| 7 | 13.2 | 13.2 | 32 | 28.1 | 3.8 |
| 8 | 13.9 | 18.8 | 33 | 29.1 | 2.5 |
| 9 | 16.1 | 34.9 | 34 | 29.4 | 9.7 |
| 10 | 16.5 | 9.6 | 35 | 30.1 | 1.9 |
| 11 | 16.7 | 12.2 | 36 | 30.6 | 2.1 |
| 12 | 17.5 | 4.1 | 37 | 30.8 | 2.7 |
| 13 | 18.5 | 100.0 | 38 | 31.3 | 1.6 |
| 14 | 18.8 | 23.2 | 39 | 31.8 | 2.5 |
| 15 | 19.2 | 24.5 | 40 | 32.2 | 5.5 |
| 16 | 19.5 | 5.6 | 41 | 32.8 | 3.5 |
| 17 | 19.8 | 21.9 | 42 | 33.7 | 8.2 |
| 18 | 20.2 | 55.8 | 43 | 34.0 | 6.9 |
| 19 | 21.0 | 12.7 | 44 | 34.8 | 3.3 |
| 20 | 21.3 | 41.5 | 45 | 35.5 | 1.9 |
| 21 | 22.5 | 7.7 | 46 | 36.6 | 3.9 |
| 22 | 23.0 | 43.2 | 47 | 37.6 | 4.6 |
| 23 | 23.2 | 9.9 | 48 | 38.6 | 3.1 |
| 24 | 23.8 | 2.5 | 49 | 39.6 | 2.6 |
| 25 | 24.3 | 12.0 | | | |

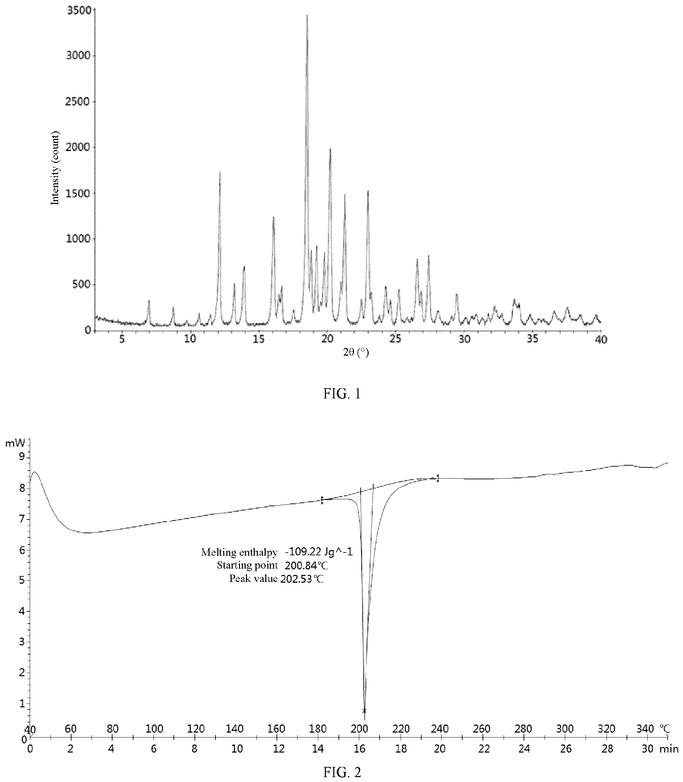

In one embodiment of the present application, crystalline form A of the compound of formula (I) has an X-ray powder diffraction pattern using Cu-Kα radiation as shown in FIG. 1.

In one embodiment of the present application, crystalline form A of the compound of formula (I) has an endothermic peak at 202.5 °C in a differential scanning calorimetry (DSC) pattern.

In one embodiment of the present application, crystalline form A of the compound of formula (I) has a starting point of an endothermic peak at 200.8 °C in a differential scanning calorimetry (DSC) pattern.

In one embodiment of the present application, crystalline form A of the compound of formula (I) has a differential scanning calorimetry (DSC) pattern as shown in FIG. 2.

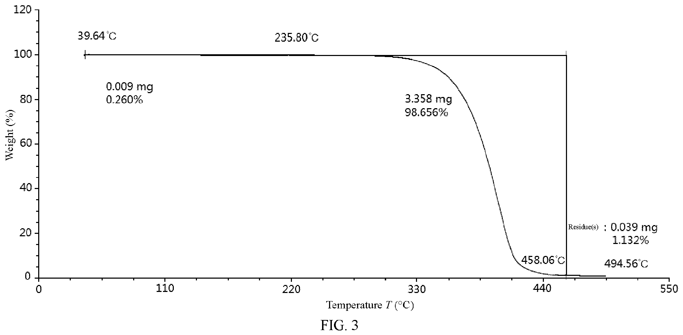

In one embodiment of the present application, crystalline form A of the compound of formula (I) has a thermogravimetric analysis (TGA) pattern as shown in FIG. 3.

In another aspect, the present application provides a method for preparing a crystalline form A of the compound of formula (I), comprising:
(a) dissolving the compound of formula (I) in a solvent to give a suspension or solution;
(b) heating the suspension or solution at reflux to give a clarified solution; and
(c) filtering the clarified solution before cooling to precipitate, filtering and drying to give crystalline form A of the compound of formula (I).

In one embodiment of the present application, the solvent in step (a) is selected from the group consisting of methanol, ethanol, isopropanol, ethyl acetate, acetonitrile, acetone, tetrahydrofuran, water, and a mixed solvent of water and any one of the above; preferably methanol, ethanol and isopropanol, and a mixed solvent thereof with water; more preferably, ethanol.

In another aspect, the present application further provides a method for preparing a crystalline form A of the compound of formula (I), comprising:
(d) dissolving the compound of formula (I) in a solvent and mixing by ultrasonication to give a suspension or solution; and
(e) heating and stirring the suspension or the solution on a thermostatic shaker, centrifuging and drying to give crystalline form A of the compound of formula (I).

In one embodiment of the present application, the solvent in step (d) is selected from the group consisting of methanol, ethanol, isopropanol, ethyl acetate, acetonitrile, acetone, tetrahydrofuran, water, and a mixed solvent of water and any one of the above; preferably, methanol, ethanol, ethyl acetate, acetonitrile, acetone, tetrahydrofuran, water, a mixed solvent of water and methanol, a mixed solvent of water and ethanol, and a mixed solvent of water and acetone. The volume ratio of water to methanol, water to ethanol, or water to acetone is selected from 1:1-1:5, preferably 1:1-1:3.

In one embodiment of the present application, the molar-to-volume ratio of the compound of formula (I) to the solvent in step (a) is 1 mmol: 1-10 mL, preferably 1 mmol:2-6 mL, more preferably 1 mmol:2-4 mL.

In one embodiment of the present application, the molar-to-volume ratio of the compound of formula (I) to the solvent in step (d) is 1 mmol:1-15 mL, preferably 1 mmol:4-10 mL, more preferably 1 mmol:8-10 mL.

In one embodiment of the present application, the reflux temperature in step (b) is 60-120 °C, preferably 80-90 °C.

In one embodiment of the present application, the stirring temperature in step (e) is 30-50 °C, preferably 40-50 °C.

In another aspect, the present application provides a crystalline composition of the compound of formula (I), comprising 50% or more, preferably 75% or more, more preferably 90% or more, and most preferably 95% or more by weight of the crystalline form of the compound of formula (I). The crystalline composition may also comprises other crystalline or amorphous forms of the compound of formula (I) in small amounts.

In another aspect, the present application provides a pharmaceutical composition, comprising a therapeutically effective amount of the crystalline form of the compound of formula (I), or the crystalline composition of the compound of formula (I); the pharmaceutical composition may further comprise at least one pharmaceutically acceptable carrier or other excipient.

In another aspect, the present application provides use of the crystalline form of the compound of formula (I), the crystalline composition of the compound of formula (I), or the pharmaceutical composition as described above in preparing a medicament for treating a cancer that can benefit from cIAP1 inhibition.

In another aspect, the present application provides the crystalline form of the compound of formula (I), the crystalline composition of the compound of formula (I), or the pharmaceutical composition as described above, for use in treating a cancer that can benefit from cIAP1 inhibition in a mammal.

In one embodiment of the present application, the mammal is a human.

In one embodiment of the application, the cancer that can benefit from cIAP1 inhibition is selected from breast cancer.

In one embodiment of the application, the cancer that can benefit from cIAP1 inhibition is selected from triple negative breast cancer.

The crystalline form of the compound of formula (I) disclosed herein has excellent effects in at least one aspect of biological activity, safety, bioavailability and the like, and crystalline form A of the compound of formula (I) features high stability, low hygroscopicity and a good inhibitory activity against cIAP1, thus having good druggability. Crystalline form A of the compound of formula (I) also demonstrates good pharmacokinetics which can be verified by preclinical (e.g., in SD rats and beagle dogs) and clinical trials, and is suitable for use as a medicament.

In the present application, the pharmaceutical composition can be formulated into a certain dosage form, and the administration route is preferably oral administration, parenteral administration (including subcutaneous, intramuscular and intravenous administration), rectal administration, and the like. For example, suitable dosage forms for oral administration include tablets, capsules, granules, pulvises, pills, powders, pastilles, syrups or suspensions; suitable dosage forms for parenteral administration include aqueous or non-aqueous solutions or emulsions for injection; suitable dosage forms for rectal administration include suppositories with hydrophilic or hydrophobic carriers.

In the present application, the X-ray powder diffraction pattern of a sample is determined in the following conditions: instrument: Bruker D8 ADVANCE X-ray diffractometer; target: Cu:Kα; wavelength λ = 1.54056 A; range of 2θ: 3-40°; scattering slit: 0.60 mm; detector slit: 10.50 mm; anti-scatter slit: 7.10 mm; step size: 0.02°; step time: 0.12 s; sample rotation speed: 15 rpm; Cu-target tube voltage and current: 40 kV, 40 mA.

In the present application, the DSC pattern is determined in the following conditions: instrument: TA Q2000 differential scanning calorimeter; temperature range: 30-300 °C; heating rate: 10 °C/min.

In the present application, TGA thermogravimetric analysis is determined in the following conditions: instrument: TA Q5000 thermogravimetric analyzer; temperature range: 25-300 °C; heating rate: 10 °C/min.

It should be noted that in the X-ray powder diffraction pattern, the position and relative intensity of a peak may vary due to measuring instruments, measuring methods/conditions, and other factors. For any specific crystalline form, the position of a peak may have an error, and the measurement of 2θ may have an error of ±0.2°. Therefore, this error should be considered when determining each crystalline form, and crystalline forms within this margin of error are within the scope of the present application.

It should be noted that, for the same crystalline form, the position of an endothermic peak in the DSC pattern may vary due to measuring instruments, measuring methods/conditions, and other factors. For any specific crystalline form, the starting point of an endothermic peak may have an error of ±5°C. Therefore, this error should be considered when determining each crystalline form, and crystalline forms within this margin of error are within the scope of the present application.

It should be noted that, for the same crystalline form, the position of a weight loss temperature in the TGA pattern may vary due to measuring instruments, measuring methods/conditions, and other factors. For any specific crystalline form, the weight loss temperature may have an error of ±5°C. Therefore, this error should be considered when determining each crystalline form, and crystalline forms within this margin of error are within the scope of the present application.

### Definitions and Description

As used in the specification and claims of the present application, the following terms, unless otherwise specified, have the meanings indicated:
"Mammal" includes human, domestic animals such as laboratory mammals and domestic pets (e.g., cat, dog, pig, cow, sheep, goat, horse, rabbit), and non-domesticated mammals such as wild mammals.

The term "pharmaceutical composition" refers to a formulation of the compound disclosed herein with a vehicle commonly recognized in the art for delivering a biologically active compound to a mammal, such as a human. The vehicle includes all pharmaceutically acceptable carriers for its use. The pharmaceutical composition facilitates administration of the compound to an organism.

The term "therapeutically effective amount" refers to an amount of a drug or a medicament that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of routine tests.

The term "pharmaceutically acceptable carriers" refers to those which are administered together with the active ingredient, do not have a significant irritating effect on an organism and do not impair the biological activity and properties of the active compound. For additional information on carriers, reference may be made to Remington: The Science and Practice of Pharmacy, 21st Ed., Lippincott, Williams & Wilkins (2005).

As used herein, "room temperature" refers to 20 °C to 30 °C.

### BRIEF DESCRIPTION OF THE DRAWINGS

FIG. 1 is an X-ray powder diffraction (XRPD) pattern of crystalline form A of the compound of formula (I) in Example 1.
FIG. 2 is a differential scanning calorimetry (DSC) pattern of crystalline form A of the compound of the formula (I) in Example 1.
FIG. 3 is a thermogravimetric analysis (TGA) pattern of crystalline form A of the compound of the formula (I) in Example 1.

### DETAILED DESCRIPTION

The following specific examples are presented to enable those skilled in the art to more clearly understand and practice the present application. These specific examples should not be considered as limit to the scope of the present application, but merely as exemplary description and representative of the present application.

The intermediate compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

The chemical reactions of the embodiments disclosed herein are carried out in a suitable solvent that must be suitable for the chemical changes in the present application and the reagents and materials required therefor. In order to acquire the compounds disclosed herein, it is sometimes necessary for those skilled in the art to modify or select a synthesis procedure or a reaction scheme based on the existing embodiments.

All solvents used in the present application are commercially available and can be used without further purification.

### Example 1: Preparation of Crystalline Form A of the Compound of Formula (I)

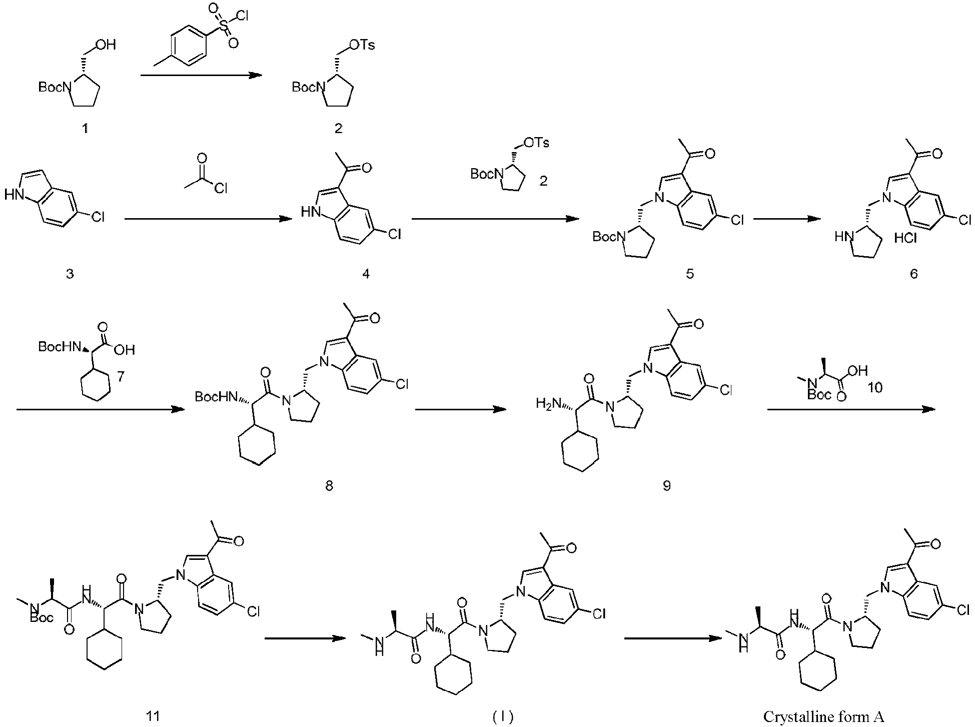

### Step 1:

Dichloromethane (16.0 L) was added to a reaction kettle and cooled to 0 °C before Compound 1 (2.0 kg, 9.94 mol, 1.0 eq) was added. The mixture was stirred and added with triethylamine (4.42 L, 29.81 mol, 3.0 eq) and 4-dimethylaminopyridine (61.00 g, 496.86 mmol, 0.05 eq). *p*-Toluenesulfonyl chloride (2.18 kg, 11.43 mol, 1.15 eq) was added in portions with the internal temperature controlled at 0-10 °C before dichloromethane (4 L) was added to the reaction kettle. The mixture was stirred for 16 h at 20 °C. Water (20 L) was added to the reaction kettle, and the mixture was stirred for 5 min, let stand and separated. The aqueous phase was removed, and the organic phase was washed with a 10% aqueous citric acid solution (15.0 L × 2) and a saline solution (15.0 L × 2), dried over anhydrous sodium sulfate (2.0 kg) for 1 h and filtered. The filtrate was concentrated by a rotary evaporator to give compound 2. ¹H NMR (400MHz, DMSO-*d₆*) δ 7.78 (br d, *J* = 8.2 Hz, 2H), 7.47 (br d, *J* = 7.9 Hz, 2H), 4.13-3.93 (m, 2H), 3.92-3.81 (m, 1H), 3.22-3.11 (m, 2H), 1.96-1.80 (m, 2H), 1.74-1.65 (m, 2H), 1.40 (s, 3H), 1.38-1.23 (m, 9H).

### Step 2:

Dichloromethane (1.30 L) and compound 3 (1.28 kg, 8.49 mol, 1.0 eq) were added into a dry reaction kettle and stirred for dissolving. The system was cooled to an internal temperature of 0 °C, and acetyl chloride (606.0 mL, 8.49 mol, 1.0 eq) was added dropwise in nitrogen atmosphere within 0.5 h. 1 M diethyl aluminum chloride in *n*-hexane (8.50 L, 8.49 mol, 1.0 eq) was then added dropwise into the reaction kettle with the internal temperature controlled at 0-5 °C within 3 h. The system was incubated for 1 h after the addition was completed. The reaction solution was slowly poured into ice water (20.0 L) in portions while stirring, and a large amount of red solid was precipitated. The quenched mixture was transferred into a rotary evaporator and concentrated at 30 °C to remove dichloromethane and *n*-hexane. 2-methyltetrahydrofuran (20.0 L) was added to the residue, and the mixture was transferred into a liquid separator, stirred for 15 min and separated to remove the aqueous phase. The organic phase was washed with a saline (20 L × 2), dried over anhydrous sodium sulfate (3.0 kg) and filtered. The filter cake was washed with 2-methyltetrahydrofuran (2 L) and the washing was filtered. The filtrate was transferred into a rotary evaporator and concentrated. The residue was added into a 50-L reaction kettle, added with ethyl acetate:n-heptane (v/v) = 1:1 (10.0 L), stirred for 10 h, and filtered. The filter cake was rinsed with ethyl acetate:n-heptane (v/v) = 1:1 (300 mL), drained, and transferred into an oven for drying at a reduced pressure at 35 °C to give compound 4. ¹H NMR (400MHz, DMSO-*d₆*) δ 12.10 (br s, 1H), 8.38 (d, *J* = 3.2 Hz, 1H), 8.16 (d, *J* = 2.2 Hz, 1H), 7.50 (d, *J* = 8.7 Hz, 1H), 7.23 (dd, *J* = 8.6, 2.1 Hz, 1H), 2.46 (s, 3H).

### Step 3:

*N*,*N*-dimethylacetamide (6.0 L) was added to a reaction kettle in nitrogen atmosphere before compound 2 (3.95 kg, 11.12 mol, 1.30 eq) and compound 4 (1.73 kg, 8.56 mol, 1.0 eq) were added at 25 °C and cesium carbonate (4.18 kg, 12.85 mol, 1.50 eq) was added in portions over 10 min. The kettle wall was flushed with *N*,*N*-diethylacetamide (1.0 L), and incubated at 80-85 °C for 16 h. The reaction solution was cooled to 25 °C, slowly added with water (30 L), added ethyl acetate (20 L), stirred and transferred to a liquid separator for separation. The water phase was extracted with ethyl acetate (10.0 L × 1). The organic phases were combined, washed with water (15.0 L × 2) and saline (15.0 L × 2), dried over anhydrous sodium sulfate (3.0 kg) for 1 h and filtered. The filter cake was rinsed with ethyl acetate (2.0 L), and the filtrate was transferred into a rotary evaporator for concentrating to give a crude product. The crude product was transferred to a reaction kettle, added with ethyl acetate:n-heptane (v/v) = 1:2 (9.0 L) stirred for 2 h and filtered. The filter cake was purified with ethyl acetate:n-heptane (v/v) = 1:2 (500 mL), drained, transferred into an oven, and dried at a reduced pressure and 35 °C to give compound 5. ¹H NMR (400MHz, DMSO-*d₆*) δ 8.43-8.28 (m, 1H), 8.17 (s, 1H), 7.74-7.52 (m, 1H), 7.27 (br s, 1H), 4.40-4.05 (m, 3H), 3.31-3.11 (m, 2H), 2.50-2.35 (m, 3H), 1.90-1.69 (m, 4H), 1.39 (br d, *J* = 6.7 Hz, 5H), 1.08 (br s, 4H).

### Step 4:

Ethyl acetate (16.5 L) was added to a dry reaction kettle before compound 5 (1.65 kg, 4.38 mol, 1.0 eq) was added. The system was stirred for dissolving and cooled to an internal temperature of 0 °C, freshly prepared hydrogen chloride in ethyl acetate (15.0 L, 4.0 mol/L) was added dropwise over 1 h. The reaction kettle was cooled to room temperature and incubated at 20-30 °C for 16 h. The reaction solution was filtered, the filter cake was rinsed with ethyl acetate (5 L × 2) and drained to give a crude product. The crude product was suspended in ethyl acetate and stirred for 4 h. The mixture was filtered and dried to give compound 6. ¹H NMR (400MHz, DMSO-*d₆*) δ 10.00 (br s, 1H), 9.54 (br s, 1H), 8.76 (s, 1H), 8.16 (d, *J* = 1.8 Hz, 1H), 7.84 (d, *J* = 8.8 Hz, 1H), 7.30 (dd, *J* = 8.7, 1.8 Hz, 1H), 4.80-4.72 (m, 1H), 4.62 (br d, *J* = 5.4 Hz, 1H), 3.92 (br s, 1H), 3.27 (br d, *J =* 6.8 Hz, 1H), 3.18-3.00 (m, 1H), 2.44 (s, 3H), 2.17-1.63 (m, 4H).

### Step 5:

Compound 7 (2.13 kg, 8.30 mol, 1.05 eq) and *N*,*N*-dimethylformamide (12.50 L) were added into a reaction kettle in sequence, and cooled to an internal temperature of 0-10 °C before benzotriazole-*N*,*N*,*N*,*N*-tetramethyluronium hexafluorophosphate (3.15 kg, 8.30 mol, 1.05 eq) was added to give a white suspension. *N*,*N*-diisopropylethylamine (3.06 kg, 23.71 mol, 3.0 eq) was dropwise added with the internal temperature controlled at 10 °C or less until the solid was gradually dissolved to give a clear solution. After the addition, the internal temperature was controlled at 5-10 °C, and the system was stirred for 0.5 h of reaction. Compound 6 (2.50 kg, 7.90 mol, 1.0 eq) was added in portions with the internal temperature controlled at 5-10 °C during which white smoke was observed. After the addition, the temperature was gradually increased to 25-30 °C before the system was incubated for 1.5 h. In stirring, half of the reaction solution was slowly dropped into a reaction kettle containing water (30.0 L). After a large amount of solid was precipitated, the mixture was stirring for 30 min and filtered, and the filter cake was drained. The other half of the reaction solution was processed by the same method. The filter cakes were combined and dried at 50 °C in vacuum to a constant weight to give compound 8. ¹H NMR (400MHz, DMSO-*d₆*) δ 8.47-8.41 (m, 1H), 8.15 (d, *J* = 2.1 Hz, 1H), 7.97-7.91 (m, 1H), 7.32 (dd, *J* = 8.7, 2.0 Hz, 1H), 6.87 (br d, *J* = 8.4 Hz, 1H), 4.49-4.30 (m, 2H), 4.15-3.89 (m, 2H), 3.62 (br dd, *J* = 8.0, 5.0 Hz, 2H), 2.44 (s, 3H), 2.17-1.97 (m, 1H), 1.96-1.85 (m, 1H), 1.78-1.52 (m, 9H), 1.38-1.31 (m, 9H), 1.20-1.06 (m, 4H).

### Step 6:

Ethyl acetate (17.70 L) was added to a 50-L reaction kettle and cooled to 0-5 °C before compound 8 (1.70 kg, 3.37 mol, 1.0 eq) was added to give a white suspension. Freshly prepared hydrogen chloride in ethyl acetate (17.70 L, 4 mol/L, 21.0 eq) was added using a peristaltic pump during which the solid was gradually dissolved to give a red-brown solution. After the addition, the temperature was gradually increased to 10-25 °C and the system was incubated for 2.5 h, and a large amount of reddish brown solid was precipitated. The reaction solution was filtered, and the filter cake and the solid on the wall of the kettle were dissolved in methanol (7.0 L). The organic phase was transferred to a rotary evaporator and concentrated at reduced pressure to give a solid. The solid was suspended in ethyl acetate (8.85 L) and stirred overnight. The mixture was filtered, and the filter cake was dried at 35-45 °C in vacuum to a constant weight to give compound 9. ¹H NMR (400MHz, DMSO-*d₆*) δ 8.50 (s, 1H), 8.30 (br d, *J* = 3.1 Hz, 3H), 8.15 (d, *J* = 2.1 Hz, 1H), 7.89 (d, *J* = 8.8 Hz, 1H), 7.31 (dd, *J* = 2.1, 8.7 Hz, 1H), 4.56-4.45 (m, 1H), 4.45-4.36 (m, 1H), 4.15 (dd, *J* = 13.8, 8.3 Hz, 1H), 3.86 (br t, *J* = 5.1 Hz, 1H), 3.70-3.62 (m, 1H), 3.61-3.56 (m, 1H), 2.44 (s, 3H), 2.19-2.03 (m, 1H), 1.96-1.87 (m, 1H), 1.83-1.66 (m, 5H), 1.60 (br d, *J* = 10.3 Hz, 3H), 1.14-0.93 (m, 4H).

### Step 7:

*N,N-*dimethylformamide (11.88 L) was added into a reaction kettle and cooled to 0-5 °C before compound 10 (1.36 kg, 6.71 mol, 1.05 eq) and benzotriazole-*N*,*N*,*N*,*N*-tetramethyluronium hexafluorophosphate (2.54 kg, 6.71 mol, 1.05 eq) were added. The solid was not completely dissolved, and thus a white suspension was obtained. *N*,*N-*diisopropylethylamine (2.48 kg, 19.16 mol and 3.0 equivalent) was added with the temperature controlled at 0-5 °C until the solid was gradually dissolved to give a clear solution. After the addition, the system was incubated at 0-5 °C for 30 min, and compound 9 (2.9 kg, 6.39 mol and 1.0 eq) was slowly added, during which a white smoke and a heat releasing phenomenon were observed. The reaction temperature was controlled at 0-5 °C by controlling the feeding speed, and after the addition, the system was incubated at 25-30 °C for 23 h. The reaction solution was processed in 5 portions. The reaction solution (about 5.0 L) was added dropwise to 35.0 L of water while stirring, and a large amount of white solid was precipitated. The mixture was filtered after stirring for 30 min, and the filter cakes were drained and combined. The filter cake and the solid on the wall of the reaction kettle were dissolved in ethyl acetate (30.0 L), dried over anhydrous sodium sulfate and filtered. The filtrate was concentrated at reduced pressure to give compound 11. 1H NMR(400MHz, CDCl₃) δ 8.34 (d, *J* = 2.0 Hz, 1H), 7.73 (s, 1H), 7.67 (br d, *J* = 8.7 Hz, 1H), 7.29-7.22 (m, 1H), 6.78 (br s, 1H), 4.78-4.45 (m, 4H), 3.88-3.55 (m, 4H), 2.84-2.76 (m, 3H), 2.50 (s, 3H), 2.06-1.94 (m, 2H), 1.73 (br d, *J* = 10.3 Hz, 6H), 1.51 (s, 9H), 1.39 (d, *J* = 6.7 Hz, 2H), 1.33 (br d, *J* = 7.0 Hz, 3H), 1.16 (br s, 4H).

### Step 8:

Ethyl acetate (10.0 L) was added into a reaction kettle and cooled to 0-5 °C. Compound 11 (3.84 kg, 6.03 mol, 1.0 eq) was dissolved in ethyl acetate (9.20 L) and added into the reaction kettle. Freshly prepared hydrogen chloride in ethyl acetate (4 mol/L, 19.20 L) was added by a peristaltic pump at a flow rate of 0.50 L/min. After the addition, the system was gradually heated through room temperature to 25-30 °C, and incubated for 19 h, and a large amount of solid was precipitated on the inner wall of the kettle. Methyl *tert*-butyl ether (10.0 L) was added to the reaction kettle. The reaction solution was stirred for 2.0 h and filtered, and the filter cake was drained. The filter cake and the solid on the kettle wall were dissolved in water (30.0 L) and cooled to 5-10 °C. The mixture was extracted twice with ethyl acetate (10.0 L × 2), and the aqueous phase was collected. The water phase was processed in two portions. 15.0 L of water was added into a reaction kettle and cooled to an internal temperature of 5-10 °C. A 10% aqueous potassium carbonate solution was dropwise added until pH 9, where the reaction solution turned colorless from mauve and a solid was precipitated. The mixture was stirred for 30 min and filtered. If the solid was too fine to filter, the mixture could be added with ethyl acetate (1.50 L) and stirred for 30 min before filtration. The filter cake was drained, transferred to the reaction kettle, added with isopropanol and stirred for 2.0 h. The mixture was filtered and the filter cake was dried in vacuum at 45 °C to a constant weight to give the compound of formula (I). ¹H NMR (400MHz, DMSO-*d₆*) δ 8.50-8.39 (m, 1H), 8.20-8.09 (m, 1H), 7.96-7.82 (m, 2H), 7.32 (dd, *J* = 8.7, 2.1 Hz, 1H), 4.58-4.34 (m, 3H), 4.14-4.02 (m, 1H), 3.79-3.54 (m, 2H), 3.06-2.88 (m, 1H), 2.48-2.41 (m, 3H), 2.24-2.13 (m, 3H), 2.09-1.95 (m, 3H), 1.93-1.85 (m, 1H), 1.79-1.54 (m, 8H), 1.12-1.01 (m, 6H), 0.99-0.86 (m, 1H).

### Step 9:

Ethanol (14.0 L) was added to the reaction kettle. The compound of formula (I) (1.74 kg, 3.41 mol, 1.0 eq) was then added to the reaction kettle while stirring. The solid was not dissolved completely, and thus a suspension was obtained. Ethanol in the kettle was refluxed in a circulating bath at 90 °C, during which the solid was completely dissolved. The mixture was then incubated for 15 min and quickly subjected to a filtration. The filtrate was transferred into the reaction kettle and gradually cooling to room temperature. A large amount of solid was precipitated. The reaction solution was filtered, and the filter cake was dried at 35-45 °C in vacuum to a constant weight to give crystalline form A of the compound of formula (I). ¹H NMR (400MHz, DMSO-*d₆*) δ 8.47-8.39 (m, 1H), 8.19-8.08 (m, 1H), 7.97-7.67 (m, 2H), 7.38-7.20 (m, 1H), 4.59-4.29 (m, 3H), 4.17-4.00 (m, 1H), 3.63 (td, *J* = 7.6, 3.8 Hz, 2H), 2.97 (q, *J* = 6.8 Hz, 1H), 2.47-2.39 (m, 3H), 2.24-2.14 (m, 3H), 2.11-1.96 (m, 2H), 1.94-1.81 (m, 1H), 1.78-1.49 (m, 8H), 1.25-0.87 (m, 8H).

### Example 2: Preparation of Crystalline Form A of the Compound of Formula (I)

Approximately 50 mg of the compound of formula (I) was transferred into 1.5mL liquid chromatograph sample vials, added with an appropriate amount of solvent or mixed solvent (see Table 2), and mixed or dissolved by sonication. The suspension samples were stirred (protected from light) on a thermostatic shaker (40 °C) for 60 h. The sample liquids were then rapidly centrifuged, and the solids were dried overnight in a vacuum drying oven at 35 °C. The dried samples were subjected to XRPD.

**Table 2: Preparation of crystalline form A of the compound of formula (I)**

| No. | Solvent or mixed solvent | Volume | State | Crystalline form |
|---|---|---|---|---|
| 1 | Methanol | 1 mL | Suspension | Crystalline form A |
| 2 | Ethanol | 1 mL | Suspension | Crystalline form A |
| 3 | Ethyl acetate | 1 mL | Suspension | Crystalline form A |
| 4 | Acetonitrile | 1 mL | Suspension | Crystalline form A |
| 5 | Acetone | 1 mL | Suspension | Crystalline form A |
| 6 | Tetrahydrofuran | 1 mL | Suspension | Crystalline form A |
| 7 | Water | 1 mL | Suspension | Crystalline form A |
| 8 | Methanol:water = 3:1 (v/v) | 1 mL | Suspension | Crystalline form A |
| 9 | Ethanol:water = 1:1 (v/v) | 1 mL | Suspension | Crystalline form A |
| 10 | Acetone:water = 1:1 (v/v) | 1 mL | Suspension | Crystalline form A |

### Experimental Example 1: Hygroscopicity Study of Crystalline Form A of the Compound of Formula (I)

### Conditions:

Instrument model: SMS DVS Advantage
Test conditions: crystalline form A of the compound of formula (I) (10-15 mg) was placed in a DVS sample tray for testing.
The DVS parameters are as follows:
   Temperature: 25 °C
   Balancing: dm/dt = 0.01%/min (shortest: 10 min, longest: 180 min)
   Drying: drying at 0% RH for 120 min
   RH (%) measurement gradient: 10 %
   RH (%) measurement gradient range: 0%-90%-0%

The hygroscopicity evaluation criteria are shown in Table 3.

**Table 3: Hygroscopicity evaluation criteria**

| Classification of hygroscopicity | Hygroscopic weight gain* |
|---|---|
| Deliquescence | Absorb sufficient water to form a |
| Very hygroscopic | ≥ 15% weight gain |
| Moderately hygroscopic | 2% to 15% weight gain |
| Slightly hygroscopic | 0.2% to 2% weight gain |
| Non-hygroscopic | < 0.2% weight gain |

| | |
|---|---|
| Note: The hygroscopic weight gain at 25 °C/80% RH. | |

Results: Crystalline form A of the compound of formula (I) demonstrated a hygroscopic weight gain of 0.72% at 25 °C and 80% RH.

Conclusion: Crystalline form A of the compound of formula (I) is slightly hygroscopic, is easy to store, and is not susceptible to deliquescence, deformation, mildew and the like, thus having good druggability.

### Experimental Example 2: Stability Test of Crystalline Form A of the Compound of Formula (I)

### Procedures:

Influencing factor experiment: 12 parts of crystalline form A of the compound of formula (I) each containing 1.50 g were prepared, with 3 parts examined in each condition. Samples were placed into open weighing bottles. For high-temperature test, the bottles were put into separate desiccators of different conditions, and then put into corresponding stability testers for inspection; for high-humidity test, the bottles were examined in stability testers for corresponding conditions.

Irradiation stability test: 4 parts of crystalline form A of the compound of formula (I) each containing 1.50 g were prepared. 2 parts were irradiation samples (one for 5-day irradiation, the other for 10-day irradiation), the other two parts were controls (one as 5-day control and the other as 10-day control). The irradiation samples were evenly spread in clean weighing bottles with no shelter, and the bottles were closed with transparent caps and put into an irradiation tester. The controls were packaged in the same manner as the irradiation samples, but enclosed with an aluminum film outside.

Long-term accelerated test: 22 parts of crystalline form A of the compound of formula (I) each containing 1.50 g were prepared. The samples were separately put into a double-layer LDPE bag with each layer of LDPE bag sealed separately. The bag was then put into an aluminum foil bag for heat sealing. At inspection time points, the corresponding test samples were taken out, covered with bottle caps. 0-day samples were thawed to room temperature and directly analyzed. Approximately 10 mg of the test article was subjected to XRPD. The compounds were placed in the following conditions and samples were taken at different time points for determining their properties, XRPD pattern, content and related substances. The conditions and test items are shown in Tables 4 and 5.

**Table 4: Detection results of crystalline form A and related substance content in influencing factor stability and irradiation stability tests**

| Conditions | Content of crystalline form A (%) | Impurity content (%) |
|---|---|---|
| Day 0 | 98.2 | 1.19 |
| 60 °C-5 days | 99 | 1.16 |
| 60 °C-10 days | 98.2 | 1.14 |
| 60 °C-30 days | 99.1 | 1.19 |
| 25 °C-92.5% RH-5 days | 99.1 | 1.16 |
| 25 °C-92.5% RH-10 days | 98.8 | 1.13 |
| 25 °C-92.5% RH-30 days | 99.2 | 1.19 |
| Irradiation-5 days | 97.8 | 1.12 |
| Irradiation-10 days | 99.3 | 1.13 |

**Table 5: Detection result of crystalline form A and related substance content in accelerated stability tests**

| Conditions | Content of crystalline form A (%) | Impurity content (%) |
|---|---|---|
| Day 0 | 98.2 | 1.19 |
| 40 °C-75% RH-1 month | 99.3 | 1.2 |
| 40 °C-75% RH-2 months | 98.1 | 1.19 |
| 40 °C-75% RH-3 months | 97.8 | 1.13 |
| 40 °C-75% RH-6 months | 98.5 | 1.12 |
| 25 °C-60% RH-3 months | 98 | 1.16 |
| 25 °C-60% RH-6 months | 99.5 | 1.12 |
| 25 °C-60% RH-9 months | 98.2 | 1.22 |
| 25 °C-60% RH-12 months | 100.2 | 1.09 |

### Conclusion:

XRPD showed that crystalline form A of the compound of formula (I) was consistent with that on day 0, suggesting that crystalline form A of the compound of formula (I) is stable in the various test conditions.

### Experimental Example 3: Binding Assay of cIAP1 BIR3 and XIAP BIR3

### Materials:

Buffer system (a buffer for cIAP1 BIR3 or XIAP BIR3): 0.1 mol/L potassium phosphate, pH 7.5, 0.1% bovine serum albumin, 0.005% Triton X-100 and 1% dimethyl sulfoxide.

Probe: ARPFAQ-K(5-FAM)-NH₂.

### Target:

clAP1-BIR3-his: RBC (Cat# APT-11-370), isolated BIR3 domain of human cIAP1 (amino acids 258-363; cIAP1 BIR3) expressed by *E*. coli as GST-fusion protein.

XIAP-BIR3-his: RBC (Cat# APT-11-374), isolated BIR3 domain of XIAP (amino acids 255-356; XIAP BIR3) expressed by *E*. coli as GST-fusion protein.

Condition: 5 nM ARPFAQ-K(5-FAM)-NH₂, 20 nM cIAP1 BIR3 and 30 nM XIAP BIR3.

### Procedures:

Firstly, fresh buffer of cIAP1 BIR3 or XIAP BIR3 was prepared, and mixed with 2 folds of cIAP1 BIR3 or XIAP BIR3 solution. A solution of test compound completely dissolved in 100% DMSO via ultrasonication was added to the buffer containing cIAP1 BIR3 or XIAP BIR3. The mixture was added with 2 folds of probes, mixed and incubated for 60 min in the dark at room temperature. Fluorescence polarization was measured and mP value was calculated, finally giving an IC₅₀ value.

The results are shown in Table 6.

### Conclusion:

The compounds of formula (I) disclosed herein exhibited good binding activity for cIAP1 BIR3 and good selectivity for cIAP1 and XIAP.

### Experimental Example 4: In vitro Cell Viability Assay

### Materials:

RPMI 1640 medium (Invitrogen-22400089), fetal bovine serum (Invitrogen-10099141), trypsin, 0.05% EDTA·4Na (Invitrogen-25300062), luminescence cell viability assay kit (Promega-G7573), Dulbecco's phosphate-buffered saline (HyClone-SH30028.01B), and 384-well plates (Corning-6007680). Envision multi-marker analyzer.

### Procedures:

1. 30 µL of MDA-MB-231 cell suspension, containing 250 MDA-MB-231 cells, was added to the wells of 384-well plates.
2. 20 µL of test compound (the test compound was subjected to a 5-fold serial dilution from 10 µM to 10^{th} concentration) was added, and then the plates were incubated in a carbon dioxide incubator for 7 days.
3. The cell plates were let stand at room temperature for 30 min.
4. 20 µL of Promega CellTiter-Glo reagent was added to each well.
5. After 10 min, the samples were detected using an Envision multi-label analyzer.

### Results: see Table 6.

### Conclusion:

The compound of the formula (I) disclosed herein has good anti-proliferative activity against MDA-MB-231 cell.

**Table 6**

| Test compound | cIAP1 BIR3 IC₅₀ (nM) | XIAP BIR3 IC₅₀ (nM) | MDA-MB-231 Cell IC₅₀ (nM) |
|---|---|---|---|
| Compound of formula (I) | 5.0 | 29.9 | 54.8 |

### Experimental Example 5: In Vivo Efficacy Study 1

*In vivo* efficacy study was conducted on BALB/c nude mice by subcutaneously implanting human tumor cell line-derived xenograft (CDX) from MDA-MB-231 triple negative breast cancer patients

### Procedures:

BALB/c nude mice, female, aged 6-8 weeks, about 18-22 g, were kept in a special pathogen-free environment and in separate ventilated cages (3 mice per cage). All cages, bedding and water were disinfected prior to use. All animals had free access to standard certified commercial laboratory diets. 48 mice were purchased from Shanghai BK Laboratory Animal Co., LTD. for the study. Each mouse was implanted subcutaneously in the right flank with cancer cells (10 × 10⁶ cells in 0.2 mL of phosphate buffered saline) for tumor growth. The mice were administered when the mean tumor volume reached about 147 mm³. The test compounds were administered orally at a daily dose of 30 mg/kg. Tumor volume was measured every 3 days with a two-dimensional caliper, and the volume was measured in mm³ and calculated according to the following formula: V = 0.5 × a × b², where a and b are the long and short diameters, respectively, of the tumor. Anti-tumor efficacy was determined by dividing the mean tumor increase volume of compound-treated animals by the mean tumor increase volume of untreated animals.

### Results: see Table 7.

### Conclusion:

In the MDA-MB-231 triple negative breast cancer CDX model for *in vivo* efficacy, the compound of formula (I) disclosed herein demonstrated better efficacy.

**Table 7**

| Examples | Dosage (mg/kg) | Tumor volume (mm³) | | | |
|---|---|---|---|---|---|
| | | Day 0 | 6 days | 13 days | 20 days |
| Blank control | 0 | 148 | 475 | 1225 | 1750 |
| Compound of formula (I) | 30 | 147 | 214 | 282 | 810 |

### Experimental Example 6: In Vivo Efficacy Study 2

*In vivo* efficacy study was conducted on BALB/c nude mice by subcutaneously implanting human tumor cell line-derived xenograft (CDX) from MDA-MB-231 triple negative breast cancer patients

### Procedures:

BALB/c nude mice, female, aged 6-8 weeks, about 18-22 g, were kept in a special pathogen-free environment and in separate ventilated cages (3 mice per cage). All cages, bedding and water were disinfected prior to use. All animals had free access to standard certified commercial laboratory diets. 48 mice were purchased from Shanghai BK Laboratory Animal Co., LTD. for the study. Each mouse was implanted subcutaneously in the right flank with tumor cells (10 × 10⁶ in 0.2 mL of phosphate buffer) for tumor growth. The mice were administered when the mean tumor volume reached about 110 mm³. The test compounds were administered orally at a daily dose of 30 mg/kg. Tumor volume was measured every 3 days with a two-dimensional caliper, and the volume was measured in mm³ and calculated according to the following formula: V = 0.5 × a × b², where a and b are the long and short diameters, respectively, of the tumor. Anti-tumor efficacy was determined by dividing the mean tumor increase volume of compound-treated animals by the mean tumor increase volume of untreated animals.

Results: see Table 8.

### Conclusion:

In the MDA-MB-231 triple negative breast cancer CDX model for *in vivo* efficacy, the compound of formula (I) disclosed herein demonstrated better efficacy.

**Table 8**

| Compound | Dosage (mg/kg) | Tumor volume (mm³) | | | |
|---|---|---|---|---|---|
| | | Day 0 | 5 days | 12 days | 20 days |
| Blank control | 0 | 110 | 261 | 526 | 772 |
| Compound of formula (I) | 30 | 110 | 111 | 86 | 177 |

## Claims

1. A crystalline form of a compound of formula (I)

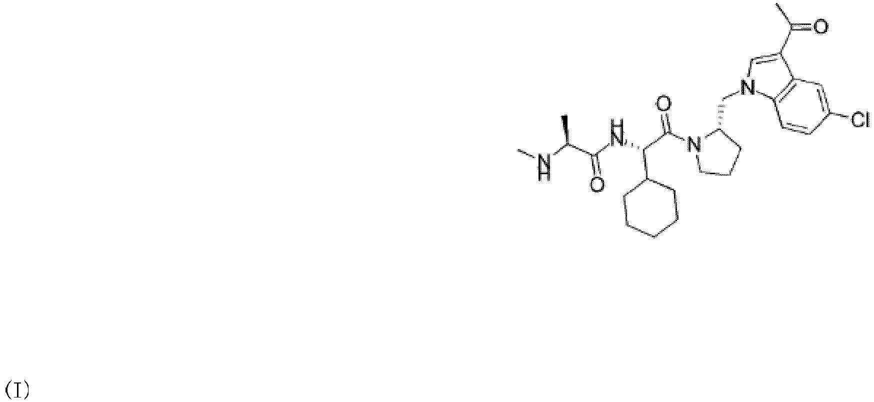

2. The crystalline form according to claim 1, wherein the crystalline form is crystalline form A of the compound of formula (I) having diffraction peaks at the following 2θ in an X-ray powder diffraction pattern using Cu-Kα radiation: 12.1°±0.200°, 16.1°±0.200°, 18.5°±0.200°, 20.2°±0.200°, 21.3°±0.200° and 23.0°±0.200°.

3. Crystalline form A of the compound of formula (I) according to claim 2, having diffraction peaks at the following 2θ in an X-ray powder diffraction pattern using Cu-Kα radiation: 12.1°±0.200°, 16.1°±0.200°, 18.5°±0.200°, 18.8°±0.200°, 19.2°±0.200°, 19.8°±0.200°, 20.2°±0.200°, 21.3°±0.200°, 23.0°±0.200°, 26.6°±0.200° and 27.4°±0.200°.

4. Crystalline form A of the compound of formula (I) according to claim 3, having diffraction peaks at the following 2θ in an X-ray powder diffraction pattern using Cu-Kα radiation: 7.0°±0.200°, 8.7°±0.200°, 12.1°±0.200°, 13.2°±0.200°, 13.9°±0.200°, 16.1°±0.200°, 16.7°±0.200°, 18.5°±0.200°, 18.8°±0.200°, 19.2°±0.200°, 19.8°±0.200°, 20.2°±0.200°, 21.0°±0.200°, 21.3°±0.200°, 23.0°±0.200°, 24.3°±0.200°, 25.3°±0.200°, 26.6°±0.200° and 27.4°±0.200°.

5. Crystalline form A of the compound of formula (I) according to claim 4, having diffraction peaks at the following 2θ in an X-ray powder diffraction pattern using Cu-Kα radiation: 7.0°±0.200°, 8.7°±0.200°, 12.1°±0.200°, 13.2°±0.200°, 13.9°±0.200°, 16.1°±0.200°, 16.5°±0.200°, 16.7°±0.200°, 18.5°±0.200°, 18.8°±0.200°, 19.2°±0.200°, 19.8°±0.200°, 20.2°±0.200°, 21.0°±0.200°, 21.3°±0.200°, 23.0°±0.200°, 23.2°±0.200°, 24.3°±9.299°, 25.3°±0.200°, 26.6°±0.200°, 26.9°±0.200°, 27.4°±0.200° and 29.4°±0.200°.

6. Crystalline form A of the compound of formula (I) according to claim 5, having diffraction peaks at the following 2θ in an X-ray powder diffraction pattern using Cu-Kα radiation: 7.0°±0.200°, 8.7°±0.200°, 9.7°±0.200°, 10.6°±0.200°, 11.4°±0.200°, 12.1°±0.200°, 13.2°±0.200°, 13.9°±0.200°, 16.1°±0.200°, 16.5°±0.200°, 16.7°±0.200°, 17.5°±0.200°, 18.5°±0.200°, 18.8°±0.200°, 19.2°±0.200°, 19.5°±0.200°, 19.8°±0.200°, 20.2°±0.200°, 21.0°±0.200°, 21.3°±0.200°, 22.5°±0.200°, 23.0°±0.200°, 23.2°±0.200°, 23.8°±0.200°, 24.3°±0.200°, 24.6°±0.200°, 25.3°±0.200°, 25.8°±0.200°, 26.6°±0.200°, 26.9°±0.200°, 27.4°±0.200°, 28.1°±0.200°, 29.1°±0.200°, 29.4°±0.200°, 30.1°±0.200°, 30.6°±0.200°, 30.8°±0.200°, 31.3°±0.200°, 31.8°±0.200°, 32.2°±0.200°, 32.8°±0.200°, 33.7°±0.200°, 34.0°±0.200°, 34.8°±0.200°, 35.5°±0.200°, 36.6°±0.200°, 37.6°±0.200°, 38.6°±0.200° and 39.6°±0.200°.

7. Crystalline form A of the compound of formula (I) according to any one of claims 2-6, having an X-ray powder diffraction pattern using Cu-Kα radiation as shown in FIG. 1.

8. Crystalline form A of the compound of formula (I) according to any one of claims 2-7, having an endothermic peak at 202.5 °C in a differential scanning calorimetry pattern.

9. Crystalline form A of the compound of formula (I) according to any one of claims 2-7, having a starting point of an endothermic peak at 200.8 °C in a differential scanning calorimetry pattern.

10. A crystalline composition, comprising 50% or more, preferably 75% or more, more preferably 90% or more, and most preferably 95% or more by weight of the crystalline form of the compound of formula (I) according to claim 1.

11. A pharmaceutical composition, comprising the crystalline form of the compound of formula (I) according to claim 1 or the crystalline composition according to claim 10.

12. The crystalline form of the compound of formula (I) according to claim 1, the crystalline composition according to claim 10, or the pharmaceutical composition according to claim 11 for use in treating a cancer that can benefit from cIAP1 inhibition.

13. The crystalline form of the compound of formula (I) according to claim 1, the crystalline composition according to claim 10, or the pharmaceutical composition according to claim 11 for the use of claim 12, wherein the cancer that can benefit from cIAP1 inhibition is selected from breast cancer; preferably selected from triple negative breast cancer.

14. A method for preparing the crystalline form A of the compound of formula (I) according to any one of claims 2-7, comprising:
(a) dissolving the compound of formula (I) in a solvent to give a suspension or solution;
(b) heating the suspension or solution at reflux to give a clarified solution; and
(c) filtering the clarified solution before cooling to precipitate, filtering and drying to give crystalline form A of the compound of formula (I);
wherein the solvent in step (a) is selected from the group consisting of methanol, ethanol, isopropanol, ethyl acetate, acetonitrile, acetone, tetrahydrofuran, water, and a mixed solvent of water and any one of the above; preferably methanol, ethanol and isopropanol, and a mixed solvent thereof with water; more preferably, ethanol.

15. A method for preparing the crystalline form A of the compound of formula (I) according to any one of claims 2-7, comprising:
(d) dissolving the compound of formula (I) in a solvent and mixing by ultrasonication to give a suspension or solution; and
(e) heating and stirring the suspension or the solution on a thermostatic shaker, centrifuging and drying to give crystalline form A of the compound of formula (I);
wherein the solvent in step (d) is selected from the group consisting of methanol, ethanol, isopropanol, ethyl acetate, acetonitrile, acetone, tetrahydrofuran, water, and a mixed solvent of water and any one of the above; preferably, methanol, ethanol, ethyl acetate, acetonitrile, acetone, tetrahydrofuran, water, a mixed solvent of water and methanol, a mixed solvent of water and ethanol, and a mixed solvent of water and acetone.

## Patentansprüche

1. Kristalline Form einer Verbindung der Formel (I)

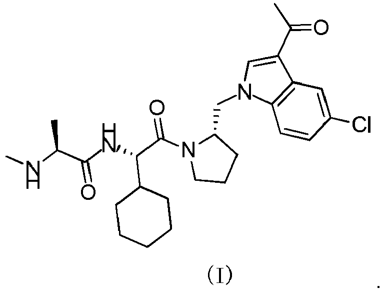

2. Kristalline Form nach Anspruch 1, wobei die kristalline Form die kristalline Form A der Verbindung der Formel (I) ist, die in einem Röntgenpulverdiffraktogramm unter Verwendung von Cu-Kα-Strahlung Beugungspeaks bei den folgenden 2θ aufweist: 12,1°±0,200°, 16,1°±0,200°, 18,5°±0,200°, 20,2°±0,200°, 21,3°±0,200° und 23,0°±0,200°.

3. Kristalline Form A der Verbindung der Formel (I) nach Anspruch 2 mit Beugungspeaks bei den folgenden 2θ in einem Röntgenpulverdiffraktogramm unter Verwendung von Cu-Kα-Strahlung: 12,1°±0,200°, 16,1°±0,200°, 18,5°±0,200°, 18,8°±0,200°, 19,2°±0,200°, 19,8°±0,200°, 20,2°±0,200°, 21,3°±0,200°, 23,0°±0,200°, 26,6°±0,200° und 27,4°±0,200°.

4. Kristalline Form A der Verbindung der Formel (I) nach Anspruch 3 mit Beugungspeaks bei den folgenden 2θ in einem Röntgenpulverdiffraktogramm unter Verwendung von Cu-Kα-Strahlung: 7.0°±0.200°, 8.7°±0.200°, 12.1°±0.200°, 13.2°±0.200°, 13.9°±0.200°, 16.1°±0.200°, 16.7°±0.200°, 18.5°±0.200°, 18.8°±0.200°, 19.2°±0. 200°, 19,8°±0,200°, 20,2°±0,200°, 21,0°±0,200°, 21,3°±0,200°, 23,0°±0,200°, 24,3°±0,200°, 25,3°±0,200°, 26,6°±0,200° und 27,4°±0,200°.

5. Kristalline Form A der Verbindung der Formel (I) nach Anspruch 4 mit Beugungspeaks bei den folgenden 2θ in einem Röntgenpulverdiffraktogramm unter Verwendung von Cu-Kα-Strahlung: 7.0°±0.200°, 8.7°±0.200°, 12.1°±0.200°, 13.2°±0.200°, 13.9°±0.200°, 16.1°±0.200°, 16.5°±0.200°, 16.7°±0.200°, 18.5°±0.200°, 18.8°±0.200°, 19.2°±0.200°, 19.8°±0. 200°, 20,2°±0,200°, 21,0°±0,200°, 21,3°±0,200°, 23,0°±0,200°, 23,2°±0,200°, 24,3°±0,200°, 25,3°±0,200°, 26,6°±0,200°, 26,9°±0,200°, 27,4°±0,200° und 29,4°±0,200°.

6. Kristalline Form A der Verbindung der Formel (I) nach Anspruch 5 mit Beugungspeaks bei den folgenden 2θ in einem R Röntgenpulverdiffraktogramm unter Verwendung von Cu-Kα-Strahlung: 7.0°±0.200°, 8.7°±0.200°, 9.7°±0.200°, 10.6°±0.200°, 11.4°±0.200°, 12.1°±0.200°, 13.2°±0.200°, 13.9°±0.200°, 16.1°±0.200°, 16.5°±0.200°, 16.7°±0.200°, 17.5°±0.200°, 18. 5°±0.200°, 18.8°±0.200°, 19.2°±0.200°, 19.5°±0.200°, 19.8°±0.200°, 20.2°±0.200°, 21.0°±0.200°, 21.3°±0.200°, 22.5°±0.200°, 23.0°±0.200°, 23.2°±0.200°, 23.8°±0.200°, 24.3°±0. 200°, 24.6°±0.200°, 25.3°±0.200°, 25.8°±0.200°, 26.6°±0.200°, 26.9°±0.200°, 27.4°±0.200°, 28.1°±0.200°, 29.1°±0.200°, 29.4°±0.200°, 30.1°±0.200°, 30.6°±0.200°, 30.8°±0. 200°, 31.3°±0.200°, 31.8°±0.200°, 32.2°±0.200°, 32.8°±0.200°, 33.7°±0.200°, 34.0°±0.200°, 34.8°±0.200°, 35.5°±0.200°, 36.6°±0.200°, 37.6°±0.200°, 38.6°±0.200° und 39.6°±0.200°.

7. Kristalline Form A der Verbindung der Formel (I) nach einem der Ansprüche 2-6 mit einem Röntgenpulverdiffraktogramm unter Verwendung von Cu-Kα-Strahlung, wie in FIG. 1 gezeigt.

8. Kristalline Form A der Verbindung der Formel (I) nach einem der Ansprüche 2-7, mit einem endothermen Peak bei 202,5 °C in einem Muster der dynamischen Differentialkalorimetrie.

9. Kristalline Form A der Verbindung der Formel (I) nach einem der Ansprüche 2-7 mit einem Ausgangspunkt für einen endothermen Peak bei 200,8 °C in einem Muster der dynamischen Differentialkalorimetrie.

10. Kristalline Zusammensetzung, die 50 Gew.-% oder mehr, vorzugsweise 75 Gew.-% oder mehr, noch bevorzugter 90 Gew.-% oder mehr und am meisten bevorzugt 95 Gew.-% oder mehr der kristallinen Form der Verbindung der Formel (I) nach Anspruch 1 enthält.

11. Pharmazeutische Zusammensetzung, umfassend die kristalline Form der Verbindung der Formel (I) nach Anspruch 1 oder die kristalline Zusammensetzung nach Anspruch 10.

12. Die kristalline Form der Verbindung der Formel (I) nach Anspruch 1, die kristalline Zusammensetzung nach Anspruch 10 oder die pharmazeutische Zusammensetzung nach Anspruch 11 zur Verwendung bei der Behandlung eines Krebses, der von einer cIAP1-Inhibition profitieren kann.

13. Die kristalline Form der Verbindung der Formel (I) nach Anspruch 1, die kristalline Zusammensetzung nach Anspruch 10 oder die pharmazeutische Zusammensetzung nach Anspruch 11 zur Verwendung nach Anspruch 12, wobei der Krebs, der von der clAP1-Inhibierung profitieren kann, aus Brustkrebs ausgewählt ist; vorzugsweise ausgewählt aus triple-negativem Brustkrebs.

14. Verfahren zur Herstellung der kristallinen Form A der Verbindung der Formel (I) nach einem der Ansprüche 2-7, umfassend:
(a) Lösen der Verbindung der Formel (I) in einem Lösungsmittel, um eine Suspension oder Lösung zu erhalten;
(b) Erhitzen der Suspension oder Lösung unter Rückfluss, um eine geklärte Lösung zu erhalten; und
(c) Filtrieren der geklärten Lösung vor dem Abkühlen zum Ausfällen, Filtrieren und Trocknen, um die kristalline Form A der Verbindung der Formel (I) zu erhalten;
wobei das Lösungsmittel in Schritt (a) ausgewählt ist aus der Gruppe bestehend aus Methanol, Ethanol, Isopropanol, Ethylacetat, Acetonitril, Aceton, Tetrahydrofuran, Wasser und einem gemischten Lösungsmittel aus Wasser und einem der vorgenannten; vorzugsweise Methanol, Ethanol und Isopropanol und ein gemischtes Lösungsmittel davon mit Wasser; besonders bevorzugt Ethanol.

15. Verfahren zur Herstellung der kristallinen Form A der Verbindung der Formel (I) nach einem der Ansprüche 2-7, umfassend:
(d) Lösen der Verbindung der Formel (I) in einem Lösungsmittel und Mischen durch Ultraschall, um eine Suspension oder Lösung zu erhalten; und
(e) Erhitzen und Rühren der Suspension oder der Lösung auf einem Thermoschüttler, Zentrifugieren und Trocknen, um die kristalline Form A der Verbindung der Formel (I) zu erhalten;
wobei das Lösungsmittel in Schritt (d) ausgewählt ist aus der Gruppe bestehend aus Methanol, Ethanol, Isopropanol, Ethylacetat, Acetonitril, Aceton, Tetrahydrofuran, Wasser und einem gemischten Lösungsmittel aus Wasser und einem der oben genannten; vorzugsweise Methanol, Ethanol, Ethylacetat, Acetonitril, Aceton, Tetrahydrofuran, Wasser, ein Lösungsmittelgemisch aus Wasser und Methanol, ein Lösungsmittelgemisch aus Wasser und Ethanol und ein Lösungsmittelgemisch aus Wasser und Aceton.

## Revendications

1. Forme cristalline d'un composé de formule (I) :

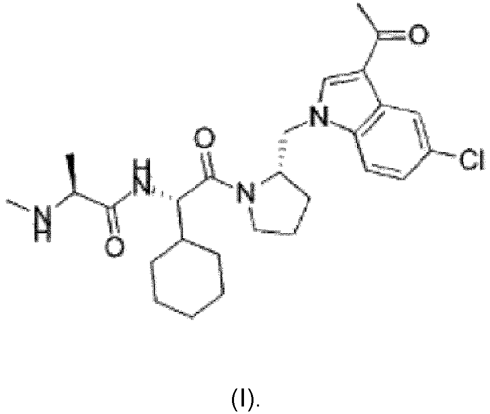

2. Forme cristalline selon la revendication 1, la forme cristalline étant la forme cristalline A du composé de formule (I) présentant des pics de diffraction aux angles 2θ suivants dans un diffractogramme de rayons X sur poudre utilisant un rayonnement Cu-Kα : 12,1° ± 0,200°, 16,1° ± 0,200°, 18,5° ± 0,200°, 20,2° ± 0,200°, 21,3° ± 0,200° et 23,0° ± 0,200°.

3. Forme cristalline A du composé de formule (I) selon la revendication 2, présentant des pics de diffraction aux angles 2θ suivants dans un diffractogramme de rayons X sur poudre utilisant un rayonnement Cu-Kα : 12,1° ± 0,200°, 16,1° ± 0,200°, 18,5° ± 0,200°, 18,8° ± 0,200°, 19,2° ± 0,200°, 19,8° ± 0,200°, 20,2° ± 0,200°, 21,3° ± 0,200°, 23,0° ± 0,200°, 26,6° ± 0,200° et 27,4° ± 0,200°.

4. Forme cristalline A du composé de formule (I) selon la revendication 3, présentant des pics de diffraction aux angles 2θ suivants dans un diffractogramme de rayons X sur poudre utilisant un rayonnement Cu-Kα : 7,0° ± 0,200°, 8,7° ± 0,200°, 12,1° ± 0,200°, 13,2° ± 0,200°, 13,9° ± 0,200°, 16,1° ± 0,200°, 16,7° ± 0,200°, 18,5° ± 0,200°, 18,8° ± 0,200°, 19,2° ± 0,200°, 19,8° ± 0,200°, 20,2° ± 0,200°, 21,0° ± 0,200°, 21,3° ± 0,200°, 23,0° ± 0,200°, 24,3° ± 0,200°, 25,3° ± 0,200°, 26,6° ± 0,200° et 27,4° ± 0,200°.

5. Forme cristalline A du composé de formule (I) selon la revendication 4, présentant des pics de diffraction aux angles 2θ suivants dans un diffractogramme de rayons X sur poudre utilisant un rayonnement Cu-Kα : 7,0° ± 0,200°, 8,7° ± 0,200°, 12,1° ± 0,200°, 13,2° ± 0,200°, 13,9° ± 0,200°, 16,1° ± 0,200°, 16,5° ± 0,200°, 16,7° ± 0,200°, 18,5° ± 0,200°, 18,8° ± 0,200°, 19,2° ± 0,200°, 19,8° ± 0,200°, 20,2° ± 0,200°, 21,0° ± 0,200°, 21,3° ± 0,200°, 23,0° ± 0,200°, 23,2° ± 0,200°, 24,3° ± 0,200°, 25,3° ± 0,200°, 26,6° ± 0,200°, 26,9° ± 0,200°, 27,4° ± 0,200° et 29,4° ± 0,200°.

6. Forme cristalline A du composé de formule (I) selon la revendication 5, présentant des pics de diffraction aux angles 2θ suivants dans un diffractogramme de rayons X sur poudre utilisant un rayonnement Cu-Kα : 7,0° ± 0,200°, 8,7° ± 0,200°, 9,7° ± 0,200°, 10,6° ± 0,200°, 11,4° ± 0,200°, 12,1° ± 0,200°, 13,2° ± 0,200°, 13,9° ± 0,200°, 16,1° ± 0,200°, 16,5° ± 0,200°, 16,7° ± 0,200°, 17,5° ± 0,200°, 18,5° ± 0,200°, 18,8° ± 0,200°, 19,2° ± 0,200°, 19,5° ± 0,200°, 19,8° ± 0,200°, 20,2° ± 0,200°, 21,0° ± 0,200°, 21,3° ± 0,200°, 22,5° ± 0,200°, 23,0° ± 0,200°, 23,2° ± 0,200°, 23,8° ± 0,200°, 24,3° ± 0,200°, 24,6° ± 0,200°, 25,3° ± 0,200°, 25,8° ± 0,200°, 26,6° ± 0,200°, 26,9° ± 0,200°, 27,4° ± 0,200°, 28,1° ± 0,200°, 29,1° ± 0,200°, 29,4° ± 0,200°, 30,1° ± 0,200°, 30,6° ± 0,200°, 30,8° ± 0,200°, 31,3° ± 0,200°, 31,8° ± 0,200°, 32,2° ± 0,200°, 32,8° ± 0,200°, 33,7° ± 0,200°, 34,0° ± 0,200°, 34,8° ± 0,200°, 35,5° ± 0,200°, 36,6° ± 0,200°, 37,6° ± 0,200°, 38,6° ± 0,200° et 39,6° ± 0,200°.

7. Forme cristalline A du composé de formule (I) selon l'une quelconque des revendications 2 à 6, ayant un diffractogramme de rayons X sur poudre utilisant un rayonnement Cu-Kα tel que présenté à la FIG. 1.

8. Forme cristalline A du composé de formule (I) selon l'une quelconque des revendications 2 à 7, présentant un pic endothermique à 202,5 °C dans un tracé d'analyse calorimétrique différentielle.

9. Forme cristalline A du composé de formule (I) selon l'une quelconque des revendications 2 à 7, présentant un point de début de pic endothermique à 200,8 °C dans un tracé d'analyse calorimétrique différentielle.

10. Composition cristalline, comprenant 50 % ou plus, de préférence 75 % ou plus, plus préférablement 90 % ou plus, et le plus préférablement 95 % ou plus en poids de la forme cristalline du composé de formule (I) selon la revendication 1.

11. Composition pharmaceutique, comprenant la forme cristalline du composé de formule (I) selon la revendication 1 ou la composition cristalline selon la revendication 10.

12. Forme cristalline du composé de formule (I) selon la revendication 1, composition cristalline selon la revendication 10, ou composition pharmaceutique selon la revendication 11 destinées à être utilisées dans le traitement d'un cancer qui peut bénéficier d'une inhibition de cIAP1.

13. Forme cristalline du composé de formule (I) selon la revendication 1, composition cristalline selon la revendication 10, ou composition pharmaceutique selon la revendication 11 destinées à être utilisées selon la revendication 12, le cancer qui peut bénéficier d'une inhibition de clAP1 étant choisi parmi un cancer du sein ; de préférence choisi parmi un cancer du sein triple négatif.

14. Procédé de préparation de la forme cristalline A du composé de formule (I) selon l'une quelconque des revendications 2 à 7, comprenant :
(a) la dissolution du composé de formule (I) dans un solvant afin de donner une suspension ou une solution ;
(b) le chauffage de la suspension ou solution sous reflux pour donner une solution clarifiée ; et
(c) le filtrage de la solution clarifiée avant de refroidir pour précipiter, un filtrage et un séchage afin de donner la forme cristalline A du composé de formule (I) ;
dans lequel le solvant de l'étape (a) est choisi dans le groupe constitué par le méthanol, l'éthanol, l'isopropanol, l'acétate d'éthyle, l'acétonitrile, l'acétone, le tétrahydrofurane, l'eau et un solvant mixte d'eau et de l'un quelconque de ceux-ci ; de préférence le méthanol, l'éthanol et l'isopropanol, et un solvant mixte de ceux-ci avec l'eau ; plus préférablement l'éthanol.

15. Procédé de préparation de la forme cristalline A du composé de formule (I) selon l'une quelconque des revendications 2 à 7, comprenant :
(d) la dissolution du composé de formule (I) dans un solvant et le mélange par sonication aux ultrasons afin de donner une suspension ou une solution ; et
(e) le chauffage et l'agitation de la suspension ou solution sur un agitateur thermostatique la centrifugation et le séchage afin de donner la forme cristalline A du composé de formule (I) ;
dans lequel le solvant de l'étape (d) est choisi dans le groupe constitué par le méthanol, l'éthanol, l'isopropanol, l'acétate d'éthyle, l'acétonitrile, l'acétone, le tétrahydrofurane, l'eau, et un solvant mixte d'eau et de l'un quelconque des précédents ; de préférence le méthanol, l'éthanol, l'isopropanol, l'acétate d'éthyle, l'acétonitrile, l'acétone, le tétrahydrofurane, l'eau, et un solvant mixte d'eau et de méthanol, un solvant mixte d'eau et d'éthanol, et un solvant mixte d'eau et d'acétone.
